# EUROPÄISCHE PATENTSCHRIFT

(11) **EP 2 432 452 B1**
(45) Veröffentlichungstag und Bekanntmachung des Hinweises auf die Patenterteilung: **27.07.2016**
(21) Anmeldenummer: 10723040.1
(22) Anmeldetag: 19.05.2010
(51) Int. Cl.: A61K 9/08, A61K 9/00, A61K 31/4184, A61K 47/10, A61K 47/26

(54) **PHARMAZEUTISCHE TELMISARTAN-TRINKLÖSUNG**
PHARMACEUTICAL TELMISARTAN DRINK SOLUTION
UNE SOLUTION PHARMACEUTIQUE À BOIRE CONTENANT TELMISARTAN

(30) Priorität: 20.05.2009 EP 09160771
(43) Veröffentlichungstag der Anmeldung: 28.03.2012
(73) Patentinhaber: Boehringer Ingelheim Vetmedica GmbH, 55216 Ingelheim am Rhein (DE)
(72) Erfinder: MOHR, Detlef, 55216 Ingelheim am Rhein (DE); LEHNER, Stefan, 55216 Ingelheim am Rhein (DE)
(74) Vertreter: Simon, Elke Anna Maria
(86) Internationale Anmeldenummer: PCT/EP2010/056895
(87) Internationale Veröffentlichungsnummer: WO 2010/133638

(56) Entgegenhaltungen:
- WO-A1-2004/028505
- US-A1- 2007 026 026

## Beschreibung

Die vorliegende Erfindung betrifft eine pharmazeutische Lösung, vorzugsweise eine pharmazeutische Trinklösung mit einem pH-Wert von 10 oder höher enthaltend einen Angiotensin II Rezeptor Antagonisten, vorzugsweise den Wirkstoff Telmisartan, sowie einen oder mehrere zur Geschmacks- und Haltbarkeitsverbesserung zugesetzte Zuckeralkohole, wobei der Gehalt an reduzierenden Zuckern vor Zugabe des oder der Zuckeralkohole zur Lösung 1000ppm nicht übersteigt.

### Hintergrund der Erfindung

Telmisartans, die INN Bezeichnung der Verbindung 4'-((2-n-Propyl-4-methyl-6-(1-methylbenz-imidazol-2-yl)-benzimidazol-1-yl)methyl)biphenyl-2-carbonsäure (IUPAC) mit folgender Formel 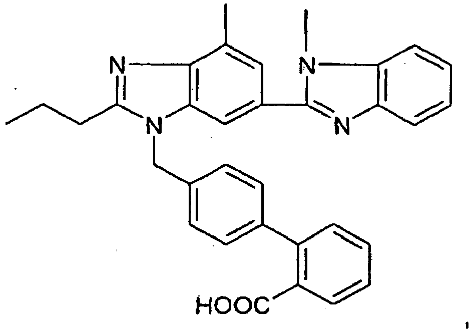 ist ein Angiotensin-II Rezeptor Antagonist, der zur Therapie der Hypertonie zugelassen und in verschiedenen Dosisstärken als Tablettenformulierung verfügbar ist. Für die Anwendung bei Kindern, insbesondere Kindern unter 6 Jahren, sowie bei älteren oder kranken Menschen mit Schluckbeschwerden ist die Anwendbarkeit von Tabletten als Arzneiform oftmals nicht möglich. Bei Haustieren wie Hunden und Katzen wäre die Anwendbarkeit von Tabletten zwar grundsätzlich möglich, sie werden jedoch häufig in Backentaschen gehalten, und später wieder ausgespuckt, was die Dosissicherheit erheblich beeinträchtigt. Zudem ist eine gewichtsadaptierte Dosierung durch die vorgegebenen fixen Dosisstufen der Tabletten allenfalls nur begrenzt möglich.

Besser geeignet, und physiologisch bevorzugt ist eine Trinklösung, frei von organischen Cosolventien, die mit Hilfe geeigneter, kalibrierter Dosierhilfsmittel wie Dosierspritzen, -pipetten, -löffeln oder -bechern in kontrollierter Menge verabreicht werden kann. Zudem besteht die Möglichkeit durch geschmacksverbessernde Zusätze die Akzeptanz der Arzneiform bei Kindern als auch bei Haustieren zu erhöhen, was insbesondere bei der chronischen Applikation im Fall der Hypertoniebehandlung vorteilhaft ist. US2007026026 offenbart eine wässrige Losartan-Zusammensetzung, WO2004028505 eine Telmisartan-Darreichungsform.

### Beschreibung der Erfindung

Aufgabe der vorliegenden Erfindung ist die Bereitstellung einer pharmazeutischen Lösung, vorzugsweise einer pharmazeutischen Trinklösung, enthaltend einen Angiotensin II Rezeptor Antagonisten laut Anspruch 1. Bevorzugter Angiotensin II Rezeptor Antagonist ist Telmisartan, sowie dessen pharmazeutisch verträglichen Salze, Hydrate oder Polymorphe. Die für die Erreichung therapeutischer Dosen notwendige Lösungskonzentration des Angiotensin II Rezeptors, vorzugsweise von Telmisartan sollte zwischen 1 mg/ml und 10 mg/ml betragen.

Aufgrund des streng pH-abhängigen Löslichkeitsprofils von Telmisartan sollte eine wässrige Formulierung entweder einen pH-Wert kleiner 2 oder von 10 oder größer 10 aufweisen, um eine physikalisch stabile Lösung zu gewährleisten. Zunächst wurde basierend auf diesen Vorgaben nach einer Formulierung im sauren pH-Bereich gearbeitet, denn, falls erforderlich (der Einsatz von Mehrdosen-behältnissen für diesen Anwendungszweck ist klar bevorzugt), sind für diesen pH-Bereich deutlich mehr Konservierungsmittel verfügbar.

Folgende prinzipielle Rezepturbestandteile kamen zur Anwendung
a) pH-aktive Komponenten für die Sicherstellung der Löslichkeit im angestrebten Konzentrationsbereich;
b) geschmackskorrigierende Komponenten (Zucker, Zuckeralkohole, Zuckerersatzstoffe, Aromazusätze)zur Überlagerung des bitteren, kratzigen Geschmacks von Telmisartan;
c) Texturverbessernde, in der Regel die Viskosität erhöhende Hilfsstoffe wie zum Beispiel Cellulosederivate, PVP, Glycerol;
d) Konservierungsmittel zur Sicherstellung der mikrobiologischen Qualität im Mehrdosenbehältnis
e) Gegebenenfalls Antioxidantien wie zum Beispiel BHA, BHT, EDTA oder Propylgallat zur Stabilisierung der Formulierung.

Bei diesen Entwicklungsarbeiten wurde überraschend festgestellt, dass für Wirkstoffkonzentrationen größer 1 mg/ml ein pH kleiner 1,5 erforderlich ist, welcher die Verwendung starker organischer Säuren wie beispielsweise Methansulfonsäure oder Phosporsäure erfordert. Die sehr stark adstringierenden Wirkung derartig saurer Lösungen macht solche sauren Formulierungen für eine chronische Anwendung insbesondere bei Kindern, aber auch bei Haustieren wie Hunden und Katzen völlig ungeeignet.

Die weiteren Entwicklungsarbeiten mussten also unerwartet auf den pH-Bereich ≥10 verlegt werden. Dazu wurden als Basenkomponenten zunächst Natronlauge, Meglumine oder eine Kombination von Natronlauge und Meglumine eingesetzt, um eine physikalisch und chemisch stabile Lösung herzustellen. Der Einsatz von Alkali- oder Erdalkalicarbonaten/-hydrogencarbonaten liefert im vorliegenden Fall keine ausreichend hohen pH-Werte. Ebenso wurde der Einsatz von Ethanolaminen hinsichtlich Applikationsweg und Zielgruppe ausgeschlossen.

Für die Überdeckung oder Verbesserung des bitteren, kratzenden Geschmacks des Telmisartans in Lösung wurden neben üblichen Mengen synthetischer Zuckeraustauschstoffe (Saccharin, Saccharin-Na, Na-Cyclamat, Acesulfam, Aspartam, Sucralose etc.), welche ein Verträglichkeitsrisiko beinhalten bzw. zum Teil nicht international zulassungsfähig sind (Na-Cyclamat), Zuckeralkohole wie zum Beispiel Xylitol, Maltitol, Sorbitol, oder Mannitol auf ihre Eignung untersucht. Sie sind nicht kariogen (langsame Umwandlung zur Säure) und haben nur einen geringen kalorischen Brennwert, was für die chronische Anwendung besonders bei Kindern aber auch bei Haustieren ein entscheidender Vorteil ist. Obwohl prinzipiell geeignet, wurde von einem Einsatz der klassischen Zucker (Glukose, Glukosesirup, Fruktose, Saccharose, Maltose, Laktose etc.) aufgrund der vorgenannten Nachteile (Kariogenität, Kalorienzufuhr) abgesehen.

Vor dem Hintergrund der Anwendungszielgruppen und der chronischen Applikation wurde wegen des Allergisierungspotentials auf den Zusatz von Aromastoffen weitgehend verzichtet, wenngleich ein Zusatz prinzipiell möglich ist, ohne die Eigenschaften der erfindungsgemäßen Telmisartanlösung zu beeinflussen. Beispiele für solche Aromastoffe sind Kirsch-, Erdbeer-, Himbeer-, Tutti-Frutti-, Johannisbeer-, Caramel-, Schokoladen- und Mintaromen, sowie Fleisch- und Fischaromen für Tiere.

Zur Verbesserung des subjektiven Gesamfeindrucks der erfindungsgemäßen Telmisartanlösung (Fülle/ Konsistenz/ Viskosität/ Textur) kann auch Glycerol sowie Cellulosederivate wie Hydroxyethylcellulose, Hydroxypropylcellulose, Methylcellulose oder Hydroxypropylmethylcellulose; oder lösliches PVP in geringeren Konzentrationen verwendet werden. Ihr Zusatz ist zwar nicht notwendig aber hilfreich, die Kontaktzeiten bis zum Erreichen der Geschmacksrezeptoren zu verlängern, und damit den bitteren Geschmack weiter zu kaschieren.

Konservierungsmittel in geeigneten Konzentrationen sind für den pH-Bereich 10 und höher nur sehr wenige verfügbar: So sind neben den Phenolderivaten wie z.B. den Kresolen, die vorwiegend parenteral im Bereich der Insulinformulierung zum Einsatz kommen, nur die quarternären Ammoniumverbindungen verfügbar. Deren Toxizität ist mit Blick auf die Zielgruppen und die chronische Verabreichung kritisch einzuschätzen. Somit ist im Grunde nur Benzalkoniumchlorid als hinreichend verträgliche Option verfügbar, Sein oral unangenehm bitterer Geschmack stellt allerdings ein zusätzliches Problem dar. Daher wurde zunächst untersucht, in welchem Grad die Konzentration der eingesetzten Zuckeralkohole die in der folgenden Tabelle zusammengefassten Pharmakopö-Anforderungen hinsichtlich Konservierungsbelastungstest (KBT) zu erfüllen vermag.

| | Geforderte Keimzahlreduktion in log KBE pro Testkeim Konservierungsbelastungstest nach USP bzw. Ph.Eur. | | | |
|---|---|---|---|---|
| | Nach 14 Tagen Inkubation | | Nach 28 Tagen Inkubation | |
| | USP | Ph.Eur. | USP | Ph.Eur. |
| Bakterien* | 1 | 3 | kein Anstieg | kein Anstieg |
| Pilze** | kein Anstieg | 1 | kein Anstieg | kein Anstieg |

| | | | | |
|---|---|---|---|---|
| * E.coli, S.aureus, P.aeruginosa ** C.albicans, A.niger | | | | |

Aufgrund ihrer Süsskraft und geschmacklichen Ähnlichkeit der Süssempfindung zum Rohrzucker sind vor allem die Zuckeralkohole Xylitol, Maltitol, Sorbitol und Mannitol bevorzugt. Besonders bevorzugt is Maltitol. Identische Ergebnisse hinsichtlich konservierender Wirkung lassen sich jedoch auch mit den anderen Zuckeralkoholen erreichen. Überraschenderweise wurde gefunden, dass ab einer Zuckeralkoholkonzentration von wenigstens 60% (GewichtsNolums-Prozent) in einer wässrigen Telmisartan-Trinklösung die Anforderungen der USP Pharmakopöe an einen Konservierungsbelastungstest erfüllt werden, nicht aber die erhöhten Anforderungen der europäischen Pharmakopöe. Dabei nimmt die zuverlässige Erreichung der Kriterien des USP-Konservierungsbelastungstests mit zunehmender Wirkstoffkonzentration ab, kann aber bis zu einer Wirkstoffkonzentration von 4 mg/ml gezeigt werden.

Es wurde nun überraschend gefunden, dass die zuverlässige Erreichung der Kriterien des USP-Konservierungsbelastungstests neben der Telmisartankonzentration und der Zuckeralkoholkonzentration von der Qualität des bzw. der eingesetzten Zuckeralkohole abhängig ist. Von besonderer Bedeutung ist hier die Menge der enthaltenen reduzierenden Zucker. Deren Spezifikation ist nach Pharmakopöenmonographie bis zu 0,2% erlaubt. Im Rahmen unserer Untersuchungen hat sich gezeigt, dass für eine sicheres Erreichen des USP-Konservierungsbelastungstests der Gehalt an reduzierenden Zucker (im Wesentlichen Maltose und Glukose) im Zuckaralkohol, d.h. die Menge reduzierender Zucker im Zuckeralkohol, vor Zugabe 1000 ppm, am besten 300 ppm nicht überschreiten sollte.

Der Grenzwert von weniger als 300 ppm reduzierender Zucker im Maltitol entspricht weniger als 300 mg reduzierender Zucker pro kg Maltitol beziehungsweise weniger als 0,3 mg reduzierender Zucker pro g Maltitol.

Eine 60%ige Zuckeralkohollösung (d.h. 60 g Zuckeralkolhol pro 100 ml Lösung) darf somit maximal 18 mg reduzierenden Zucker pro 100 ml beziehungsweise 0,18 mg reduzierenden Zucker pro ml Trinklösung enthalten.

Der Grenzwert bedeutet zum Beispiel für eine
- 40%ige Zuckeralkohollösung weniger als 0,12 mg reduzierender Zucker pro ml Trinklösung;
- 50%ige Zuckeralkohollösung weniger als 0.15 mg reduzierender Zucker pro ml Trinklösung;
- 60%ige Zuckeralkohollösung weniger als 0,18 mg reduzierender Zucker pro ml Trinklösung; oder für eine
- 70%ige Zuckeralkohollösung weniger als 0,21 mg reduzierender Zucker pro ml Trinktösung

Bezogen auf die Wirkstoffmenge des Angiotensin II Rezeptor Antagonisten, vorzugsweise Telmisartans, sollte der Gehalt an reduzierenden Zuckern weniger als 20 Gewichtsprozent betragen, bevorzugt weniger als 10 Gewichtsprozent und besonders bevorzugt weniger als 5 Gewichtsprozent.

Überraschenderweise konnte also gezeigt werden, dass die Stabilität einer Telmisartan-Trinklösung von der Menge der im Zuckeralkohol enthaltenen reduzierenden Zucker abhängig ist. So wird die Stabilität einer Telmisartan-Trinklösung mit steigendem Gehalt an reduzierenden Zuckern schlechter. Es kommt zur Bildung saurer Abbauprodukte und einem daraus resultierenden pH-Abfall innerhalb der Lösung, der schließlich zur Ausfällung des Telmisartans führt, sobald der pH unter 9,5/9.0 abfällt. Um eine lagerstabile Telmisartanlösung zu erhalten, muss aber auf den Zusatz von Zuckeralkoholen wie z.B. Xylitol, Sorbitol oder Maltitol nicht verzichtet werden, sofern die Menge an reduzierenden Zuckern im Zuckeralkohol einer wässrigen Telmisartan-Trinklösung mit einem pH von ≥ 10 oder höher unter die Pharmakopöen-Spezifikation für Zuckeralkohole limitiert wird. Bevorzugt ist ein Grenzwert von 300 ppm oder weniger. Eine entsprechend hergestellte wässrige Telmisartan-Trinklösung ist langzeitstabil. Sie besitzt somit eine Lagerstabilität von mindestens 12 Monaten bei 25°C/60% RH und 30°C/70% RH, vorzugsweise mindestens 36 Monate bei 25°C/60% RH und 30°C/70% RH. Allerdings hat sich auch herausgestellt, dass eine Menge an reduzierenden Zuckern von mindestens 250 ppm die Stabilität des Wirkstoffs positiv beeinflusst. Von daher ist ein Gehalt zwischen 1000 ppm, vorzugsweise 300 ppm und 250 ppm an reduzierenden Zuckern in der pharmazeutischen Lösung als bevorzugt anzusehen.

Eine unter Verwendung eines Zuckeralkohols mit 300 ppm oder weniger Anteil an reduzierendem Zucker hergestellte Telmisartan-Trinklösung benötigt keine Antioxidantien oder Stabilisatoren, sodass deren Zusatz optional ist.

Da durch den ausschließlichen Einsatz eines oder mehrerer Zuckeralkohole nur die USP Anforderung auf ausreichende Konservierung erfüllt werden, wurde für eine internationale Zulassung beispielhaft der Zusatz von Benzalkoniumchlorid als Konservierungsmittel untersucht. Optimiert wurde dabei entsprechend der Vorgaben durch die Pharmakopöen auf den geringstmöglichen Zusatz zur Erreichung der Kriterien. Es konnte gezeigt werden, das wässrige Telmisartan-Trinklösungen im Konzentrationsbereich 1-10 mg/ml, vorzugsweise 1-5 mg/ml, mit einem pH-Wert von ≥ 10 oder höher, einem Zuckeralkoholanteil von 40 Gewichts/Volums-Prozent (% G/V) oder mehr sowie weiteren optionalen Formulierungshilfsstoffen ab einer Benzalkoniumchloridkonzentration von 0,005% G/V oder höher zuverlässig auch die Anforderungen der Ph.Eur. erfüllen wobei der bittere Geschmack des Benzalkoniumchlorid zuverlässig überdeckt werden kann. Damit wird einerseits die Einhaltung der Vorgaben der Ph.Eur. gewährleistet, andererseits wird die physiologische Belastung auf ein Minimum reduziert.

### Herstellverfahren

Die vorliegende Erfindung beschreibt auch ein Verfahren zur Herstellung der erfindungsgemäßen Telmisartan-Trinklösung enthaltend 1-10 mg/ml Telmisartan, bevorzugt 1-5 mg/ ml Telmisartan. Soweit möglich nutzt dieses Verfahren auch aus der Literatur bekannte Verfahren für wässrige Oralialösungen.

Das Verfahren ist dadurch gekennzeichnet, das man eine geeignete Menge Wirkstoff, gegebenenfalls in Form eines Salzes oder Hydrates, mit einem molaren Überschuss einer physiologisch verträglichen Base gelöst in Wasser zusammenbringt, um eine Lösung mit einen pH von ≥ 10 zu erhalten. Weitere Formulierungshilfsmittel werden gegebenfalls zugegeben, und es wird mit gereinigtem Wasser derart auffüllt, dass sich ein Konzentrationsbereich von 1-10 mg Wirkstoff pro Milliliter Lösung ergibt. Es ist zu beachten, dass die Lösung den bereits angeführten Eigenschaften insbesondere der des pH-Wertes von ≥ 10 entspricht.

In eine besonderen Ausführungsform wird eine konsistenzgebende Komponente wie zum Beispiel ein Cellulosederivat oder PVP in einer vorgegebenen Menge gereinigtem Wasser bei Raumtemperatur vorgequollen, anschließend zur vollständigen Auflösung auf eine Temperatur von 70-80°C erwärmt und danach durch Zugabe der physiologisch verträglichen Base ein pH von 10 eingestellt, wobei die Temperatur auf 70-80°C gehalten wird. Anschliessend erfolgt unter Rühren die Zugabe des Wirkstoffes bis zum Erreichen einer Konzentration von 1-10 mg/ml, sowie die Zugabe und Auflösung des Zuckeralkohols mit einem Gehalt von weniger als 1000ppm, bevorzugt weniger als 300 ppm an reduzierendem Zucker. Nach Zugabe und Auflösung des Zuckeralkohols wird die Lösung auf Raumtemperatur abgekühlt. Weitere Formulierungshilfstoffe, wie synthetische Zuckeraustauschstoffe, Aromen oder Konservierungsmittel werden unter Rühren bei RT hinzugefügt. Abschließend wird der pH-Wert mit der physiologisch verträglichen Base wieder auf pH 10 eingestellt, bevor mit gereinigtem Wasser auf das Sollgewicht/-volumen aufgefüllt wird. Final kann die Lösung zu Entfernung partikulärer Verunreinigungen filtriert werden, bevor sie in geeignete Glas- oder Kunststoffflaschen abgefüllt wird. Die erfindungsgemäße Telmisartan-Trinklösung zeigt eine hohe Lagerstabilität, die weder durch physikalische Instabilitäten noch durch Wirkstoffabbaureaktionen begrenzt ist, und mit Hinblick auf den eingestellten pH-Wert zeigt sie eine hinreichende physiologische Verträglichkeit.

Die erfindungsgemäße Telmisartan-Trinklösung soll durch nachfolgende Beispiele erläutert werden. Die Beispiele dienen nur der Veranschaulichung und sind nicht als limitierend anzusehen.

### BEISPIELE

### Beispiel 1

| | |
|---|---|
| Telmisartan | 0,2 g |
| NaOH 1N | 0,68 ml |
| Maltitol* | 60,0 g |
| Hydroxyethylcellulose | 0,1 g |
| Gereinigtes Wasser | ad 100 ml |
| pH | 10 |
| Stabilität | |
| 25°C/ 60% RH | > 12 (18?) Monate |
| 30°C/ 70% RH | > 12 (18?) Monate |
| Erfüllt KBT** nach USP/ Ph.Eur. | USP |

| | |
|---|---|
| * ≤ 300 ppm red. Zucker, ** KBT = Konservierungsbelastungstest | |

### Beispiel 2

| | |
|---|---|
| Telmisartan | 0,1 g |
| Meglumine | 0,6 g |
| Maltitol* | 50,0 g |
| Saccharin-Na | 0,6 g |
| Hydroxyethylcellulose | 0,1 g |
| Gereinigtes Wasser | ad 100 ml |
| pH | 10 |
| Stabilität | |
| 25°C/ 60% RH | > 12 Monate |
| 30°C/ 70% RH | > 12 Monate |
| Erfüllt KBT** nach USP/ Ph.Eur. | --- |

| | |
|---|---|
| * ≤ 300 ppm red. Zucker, ** KBT = Konservierungsbelastungstest | |

### Beispiel 3

| | |
|---|---|
| Telmisartan | 4 mg/ ml |
| NaOH 1N | 0,8 ml |
| Maltitol* | 60,0 g |
| Hydroxyethylcellulose | 0,1 g |
| Benzalkoniumchlorid | 0,005 % |
| Gereinigtes Wasser | ad 100 ml |
| pH | 10 |
| Stabilität | |
| 25°C/ 60% RH | > 24 Monate |
| 30°C/ 70% RH | > 24 Monate |
| Erfüllt KBT** nach USP/ Ph.Eur. | USP und Ph.Eur. |

| | |
|---|---|
| * ≤ 300 ppm red. Zucker, ** KBT = Konservierungsbelastungstest | |

### Beispiel 4

| | |
|---|---|
| Telmisartan | 0,40 kg/ 100 L |
| NaOH 1N | 0,8 kg/ 100 L |
| Maltitol* | 60,0 kg/ 100 L |
| Hydroxyethylcellulose | 0,10 kg/ 100 L |
| Benzalkoniumchlorid | 0,01 kg/ 100 L% |
| Gereinigtes Wasser | ad 100 L |
| pH | 10 |
| Stabilität | |
| 25°C/ 60% RH | > 24 Monate |
| 30°C/ 70% RH | > 24 Monate |
| Erfüllt KBT** nach USP/ Ph.Eur. | USP und Ph.Eur. |

| | |
|---|---|
| * ≤ 300 ppm red. Zucker, ** KBT = Konservierungsbelastungstest | |

### Beispiel 5

| | |
|---|---|
| Telmisartan | 0,1 g |
| Meglumine | q.s. ad pH 10 |
| Maltitol* | 70,0 g |
| Kollidon K 25 | 0,2 g |
| Gereinigtes Wasser | ad 100 ml |
| pH | 10 |
| Stabilität | |
| 25°C/ 60% RH | > 18 (12?) Monate |
| 30°C/ 70% RH | > 18 (12?) Monate |
| Erfüllt KBT** nach USP/ Ph.Eur. | USP |

| | |
|---|---|
| * ≤ 300 ppm red. Zucker, ** KBT = Konservierungsbelastungstest | |

### Beispiel 6

| | |
|---|---|
| Telmisartan | 4 mg/ml |
| NaOH 1N | 0,8 ml |
| Xylitol* | 60,0 g |
| Hypromellose | 0,1 g |
| Gereinigtes Wasser | ad 100 ml |
| pH | 10 |
| Stabilität | |
| 25°C/60% RH | > 12 (18?) Monate |
| 30°C/ 70% RH | > 12 (18?) Monate |
| Erfüllt KBT** nach USP/ Ph.Eur. | USP |

| | |
|---|---|
| * ≤ 300 ppm red. Zucker, ** KBT = Konservierungsbelastungstest | |

### Biespiel 7

| | |
|---|---|
| Telmisartan | 2 mg/ml |
| NaOH 1N | 0,68 ml |
| Sorbitol | 60,0 g |
| Hypromellose | 0,1 g |
| Gereinigtes Wasser | ad 100 ml |
| pH | 10 |
| Stabilität | |
| 25°C/ 60% RH | > 18 Monate |
| 30°C/ 70% RH | > 18 Monate |
| Erfüllt KBT** nach USP/ Ph.Eur. | USP |

| | |
|---|---|
| * ≤ 300 ppm red. Zucker, ** KBT = Konservierungsbelastungstest | |

### Beispiel 8

| | |
|---|---|
| Telmisartan (5 mg/ml) | 0,5000 g |
| NaOH | 1,9000 g |
| Maltitol* | 60,000 g |
| Hydroxyethylcellulose | 0,1000 g |
| Gereinigtes Wasser | 59,1600 g |
| pH | 10 |
| Stabilität | |
| 25°C/ 60% RH | > 24 Monate |
| 30°C/ 70% RH | > 24 Monate |
| Erfüllt KBT** nach USP/ Ph.Eur. | USP |

| | |
|---|---|
| * ≤ 300 ppm red. Zucker, ** KBT = Konservierungsbelastungstest | |

## Patentansprüche

1. Pharmazeutische Lösung mit einem pH-Wert von 10 oder höher enthaltend Telmisartan, **dadurch gekennzeichnet, dass** ein oder mehrere Zuckeralkohole bis zu einer Gesamtkonzentration von 40 Gew% bis 70 Gew%, bevorzugt 60 Gew% bis 70 Gew%, enthalten sind, wobei der oder die Zuckeralkohole vor ihrer Zugabe zur Lösung einen Maximalgehalt von 1000 ppm an reduzierendem Zucker aufweisen.

2. Die pharmazeutische Lösung nach Anspruch 1, **dadurch gekennzeichnet, dass** es sich bei der Lösung um eine Trinklösung handelt.

3. Die pharmazeutische Lösung nach einem der Ansprüche 1 oder 2, **dadurch gekennzeichnet, dass** die pharmazeutische Lösung einen Maximalgehalt von ≥ 1000 ppm an reduzierenden Zuckern aufweist.

4. Die pharmazeutische Lösung nach einem der Ansprüche 1 bis 3, **dadurch gekennzeichnet, dass** die pharmazeutische Lösung einen Minimalgehalt von 250 ppm an reduzierenden Zuckern aufweist.

5. Die pharmazeutische L ösung nach einem der Ansprüche 1 bis 4, **dadurch gekennzeichnet, dass** der pH-Wert durch die Gegenwart von Meglumin, Natriumhydroxid, Kaliumhydroxid oder einer basischen Aminosäure wie Arginin oder Glutamin erreicht wird.

6. Die pharmazeutische L ösung nach einem der Ansprüche 1 bis 5, **dadurch gekennzeichnet dass** Telmisartan in einer Konzentration von 1 bis 10 mg/ml bevorzugt 1 bis 5 mg/ml vorliegt.

7. Die pharmazeutische Lösung nach einem der Ansprüche 1 bis 6, **dadurch gekennzeichnet, dass** der Anteil an reduzierenden Zuckern in der Lösung bezogen auf die Wirkstoffmenge des Telmisartans weniger als 20 Gewichtsprozent beträgt.

8. Die pharmazeutische Lösung nach einem der Ansprüche 1 bis 7, **dadurch gekennzeichnet, dass** der oder die Zuckeralkohole ausgewählt sind aus der Gruppe Xylitol, Maltitol, Sorbitol, Mannitol, Erythrit und Isomalt.

9. Die pharmazeutische Lösung nach einem der Ansprüche 1 bis 8, **dadurch gekennzeichnet dass** sie gegebenenfalls weitere Formulierungshilfsstoffe enthält.

10. Die pharmazeutische Lösung nach einem der Ansprüche 1 bis 9, **dadurch gekennzeichnet, dass** gegebenenfalls o-Kresol, m-Kresol, p-Kresol, oder Benzalkoniumchlorid als Konservierungsmittel allein oder in Kombination zugesetzt sind.

11. Die pharmazeutische Lösung nach einem der Ansprüche 1 bis 10, **dadurch gekennzeichnet, dass** sie weitestgehend frei ist, vorzugsweise frei ist von Antioxidantien oder Stabilisatoren.

12. Die pharmazeutische Lösung nach einem der Ansprüche 1 bis 11 zur Verwendung bei der Behandlung einer Hypertonie oder von Nierenleiden bei Menschen, insbesondere Kindern, alten Menschen oder Menschen mit Schluckbeschwerden; oder bei Tieren, insbesondere Hunden oder Katzen.

13. Methode zur Herstellung einer pharmazeutische Lösung nach einem der Ansprüche 1 bis 11, **gekennzeichnet durch**
a) Auflösen von Telmisartan und einem Zuckeralkohol mit einem Gehalt an reduzierenden Zuckern von weniger als 1000ppm, bevorzugt weniger als 300ppm, in einem Überschuss einer pharmazeutisch verträglichen Base bis sich ein pH-Wert von mindestens 10 oder höher einstellt; und
b) Einstellen der Konzentration von Telmisartan auf 1 bis 10 mg/ml.

14. Methode gemäß Anspruch 13 enthaltend die Schritte:
a) Verquellen eines konsistenzgebenden Mittels mit gereinigtem Wasser;
b) Auflösen des verquollenen konsistenzgebenden Mittels bei einer Temperatur von ≥ 50°C;
c) Zugabe einer pharmazeutisch verträglichen Base bei einer Temperatur von ≥ 50°C bis sich ein pH-Wert von ≥ 10,0 einstellt;
d) Zugabe und Auflösen des Telmisartans und des Zuckeralkohols bei einer Temperatur von ≥ 50°C;
e) Abkühlen der Lösung auf Raumtemperatur; und
f) Auffüllen der wässrigen Lösung mit gereinigtem Wasser auf die Sollkonzentration des Telmisartans.

15. Methode nach Anspruch 14, wobei die Schritte b) bis d) bei einer Temperatur zwischen 70°C und 80°C durchgeführt werden.

16. Methode nach einem der Ansprüche 14 oder 15, wobei das Verfahren den folgenden zusätzlichen Schritt umfasst:
g) Zugabe von einem oder mehreren synthetischen Zuckeraustauschstoffen, Aromen und/oder Konservierungsmittel unter Rühren bei Raumtemperatur.

17. Methode nach Anspruch 16, wobei der zusätzliche Schritt g) zwischen den Schritten e) und f) durchgeführt wird.

18. Methode nach Anspruch 13, **dadurch gekennzeichnet, dass** die pharmazeutische Lösung zur Behandlung einer Hypertonie oder von Nierenleiden bei Menschen oder Tieren hergerichtet wird.

19. Glas- oder Kunststoffbehältnis mit oder ohne Dosierhilfsmittel enthaltend die Trinklösung nach einem der Ansprüche 1 bis 12.

## Claims

1. Pharmaceutical solution with a pH value of 10 or higher, containing telmisartan, **characterised in that** one or more sugar alcohols are present up to a total concentration of 40 wt.% to 70 wt.%, preferably 60 wt.% to 70 wt.%, the sugar alcohol or alcohols having a maximum content of 1000 ppm of reducing sugar before they are added to the solution.

2. The pharmaceutical solution according to claim 1, **characterised in that** the solution is a drinkable solution.

3. The pharmaceutical solution according to claim 1 or 2, **characterised in that** the pharmaceutical solution has a maximum content of 1000 ppm of reducing sugars.

4. The pharmaceutical solution according to one of claims 1 to 3, **characterised in that** the pharmaceutical solution has a minimum content of 250 ppm of reducing sugars.

5. The pharmaceutical solution according to one of claims 1 to 4, **characterised in that** the pH is obtained by the presence of meglumine, sodium hydroxide, potassium hydroxide or a basic amino acid such as arginine or glutamine.

6. The pharmaceutical solution according to one of claims 1 to 5, **characterised in that** telmisartan is present in a concentration of 1 to 10 mg/ml, preferably 1 to 5 mg/ml.

7. The pharmaceutical solution according to one of claims 1 to 6, **characterised in that** the proportion of reducing sugars in the solution based on the quantity of active substance of the telmisartan is less than 20 % by weight.

8. The pharmaceutical solution according to one of claims 1 to 7, **characterised in that** the sugar alcohol or alcohols are selected from among xylitol, maltitol, sorbitol, mannitol, erythritol and isomaltol.

9. The pharmaceutical solution according to one of claims 1 to 8, **characterised in that** it optionally contains other formulation adjuvants.

10. The pharmaceutical solution according to one of claims 1 to 9, **characterised in that** optionally o-cresol, m-cresol, p-cresol or benzalkonium chloride are added as preservatives, alone or in combination.

11. The pharmaceutical solution according to one of claims 1 to 10, **characterised in that** it is substantially free, preferably free from antioxidants or stabilisers.

12. The pharmaceutical solution according to one of claims 1 to 11 for use in treating hypertension or kidney disease in people, particularly children, old people or people having difficulty swallowing; or in animals, particularly dogs or cats.

13. Method of preparing a pharmaceutical solution according to one of claims 1 to 11, **characterised by**
a) dissolving telmisartan and a sugar alcohol having a content of reducing sugars of less than 1000 ppm, preferably less than 300 ppm, in an excess of a pharmaceutically acceptable base until a pH of at least 10 or higher is obtained; and
b) adjusting the concentration of telmisartan to 1 to 10 mg/ml.

14. Method according to claim 13, comprising the steps of:
a) swelling a consistency-producing agent with purified water;
b) dissolving the swollen consistency-producing agent at a temperature of ≥ 50°C;
c) adding a pharmaceutically acceptable base at a temperature of ≥ 50°C until a pH of ≥ 10.0 is obtained;
d) adding and dissolving the telmisartan and the sugar alcohol at a temperature of ≥ 50°C;
e) cooling the solution to ambient temperature; and
f) topping up the aqueous solution with purified water to give the desired concentration of the telmisartan.

15. Method according to claim 14, wherein steps b) to d) are carried out at a temperature of between 70°C and 80°C.

16. Method according to one of claims 14 or 15, wherein the process comprises the following additional step:
g) adding one or more synthetic sugar substitutes, flavourings and/or preservatives, with stirring, at ambient temperature.

17. Method according to claim 16, wherein the additional step g) is carried out between steps e) and f).

18. Method according to claim 13, **characterised in that** the pharmaceutical solution is intended for treating hypertension or kidney disease in humans or animals.

19. Glass or plastics container with or without dosing aid, containing the drinkable solution according to one of claims 1 to 12.

## Revendications

1. Solution pharmaceutique ayant un pH de 10 ou supérieur, contenant du telmisartan, **caractérisée en ce qu'**un ou plusieurs polyalcools glucidiques sont contenus jusqu'à une concentration totale de 40 % en poids à 70 % en poids, de préférence 60 % en poids à 70 % en poids, dans laquelle le ou les polyalcools glucidiques présentent avant leur addition dans la solution, une teneur maximale de 1 000 ppm de sucre réducteur.

2. Solution pharmaceutique selon la revendication 1, **caractérisée en ce que** la solution est une solution à boire.

3. Solution pharmaceutique selon l'une quelconque des revendications 1 ou 2, **caractérisée en ce que** la solution pharmaceutique présente une teneur maximale de 1 000 ppm de sucres réducteurs.

4. Solution pharmaceutique selon l'une quelconque des revendications 1 à 3, **caractérisée en ce que** la solution pharmaceutique présente une teneur minimale de 250 ppm de sucres réducteurs.

5. Solution pharmaceutique selon l'une quelconque des revendications 1 à 4, **caractérisée en ce que** le pH est atteint par la présence de méglumine, d'hydroxyde de sodium, d'hydroxyde de potassium ou d'un acide aminé basique tel que l'arginine ou la glutamine.

6. Solution pharmaceutique selon l'une quelconque des revendications 1 à 5, **caractérisée en ce que** le telmisartan se présente en une concentration de 1 à 10 mg/ml, de préférence de 1 à 5 mg/ml.

7. Solution pharmaceutique selon l'une quelconque des revendications 1 à 6, **caractérisée en ce que** la proportion de sucres réducteurs dans la solution par rapport à la quantité de principe actif du telmisartan est inférieure à 20 % en poids.

8. Solution pharmaceutique selon l'une quelconque des revendications 1 à 7, **caractérisée en ce que** le ou les polyalcools glucidiques sont choisis dans le groupe comprenant le xylitol, le maltitol, le sorbitol, le mannitol, l'érythritol et l'isomalt.

9. Solution pharmaceutique selon l'une quelconque des revendications 1 à 8, **caractérisée en ce qu'**elle contient éventuellement d'autres adjuvants de formulation.

10. Solution pharmaceutique selon l'une quelconque des revendications 1 à 9, **caractérisée en ce que** l'on ajoute éventuellement du o-crésol, du m-crésol, du p-crésol ou du chlorure de benzalkonium comme conservateurs, seuls ou en combinaison.

11. Solution pharmaceutique selon l'une quelconque des revendications 1 à 10, **caractérisée en ce qu'**elle est largement dépourvue, de préférence, elle est dépourvue d'antioxydants ou de stabilisants.

12. Solution pharmaceutique selon l'une quelconque des revendications 1 à 11, pour son utilisation dans le traitement d'une hypertension ou d'affections rénales chez l'être humain, en particulier chez l'enfant, les sujets âgés ou ceux qui présentent des troubles de la déglutition ; ou chez l'animal, en particulier le chien ou le chat.

13. Méthode de production d'une solution pharmaceutique selon l'une quelconque des revendications 1 à 11, **caractérisée par**
a) la dissolution de telmisartan et d'un polyalcool glucidique ayant une teneur en sucres réducteurs inférieure à 1 000 ppm, de préférence, inférieure à 300 ppm, dans un excédent d'une base pharmaceutiquement acceptable jusqu'à ce qu'un pH d'au moins 10 ou supérieur s'instaure ; et
b) l'ajustement de la concentration de telmisartan à 1 à 10 mg/ml.

14. Méthode selon la revendication 13, comprenant les étapes de :
a) gonflement d'un agent de consistance avec de l'eau purifiée ;
b) dissolution de l'agent de consistance gonflé à une température ≥ 50,0 °C ;
c) addition d'une base pharmaceutiquement acceptable à une température ≥ 50 °C jusqu'à ce qu'un pH de ≥ 10,0 s'instaure ;
d) addition et dissolution du telmisartan et du polyalcool glucidique à une température ≥ 50 °C ;
e) refroidissement de la solution à température ambiante ; et
f) instauration de la concentration théorique de telmisartan de la solution aqueuse par addition d'eau purifiée.

15. Méthode selon la revendication 14, dans laquelle les étapes b) à d) sont réalisées à une température entre 70 °C et 80 °C.

16. Méthode selon l'une quelconque des revendications 14 ou 15, dans laquelle le procédé comprend les étapes supplémentaires suivantes :
g) addition d'un ou plusieurs substituts de sucre synthétiques, d'arômes et/ou de conservateurs en agitant à température ambiante.

17. Méthode selon la revendication 16, dans laquelle l'étape g) supplémentaire est réalisée entre les étapes e) et f).

18. Méthode selon la revendication 13, **caractérisée en ce que** la solution pharmaceutique est préparée pour le traitement d'une hypertension ou d'affections rénales chez l'être humain ou l'animal.

19. Contenant en verre ou en matière plastique avec ou sans élément doseur contenant la solution à boire selon l'une quelconque des revendications 1 à 12.
